# EUROPEAN PATENT SPECIFICATION

(11) **EP 3 090 725 B1**
(45) Date of publication and mention of the grant of the patent: **27.11.2019**
(21) Application number: 16168045.9
(22) Date of filing: 03.05.2016
(51) Int. Cl.: A61K 8/31, A61K 8/34, A61K 8/37, A61K 8/60, A61Q 19/00, A61K 8/92, A61K 8/02, A61K 8/06

(54) **COSMETIC COMPOSITION AND TEXTILE PRODUCT COMPRISING SAID COMPOSITION**
KOSMETISCHE ZUSAMMENSETZUNG UND TEXTILPRODUKT ENTHALTEND DIE ZUSAMMENSETZUNG
COMPOSITION COSMÉTIQUE ET PRODUIT TEXTILE COMPRENANT LADITE COMPOSITION COSMÉTIQUE

(30) Priority: 05.05.2015 IT RM20150194
(43) Date of publication of application: 09.11.2016
(73) Proprietor: DIVA BRANDS & PATENTS S.r.l., 06034 Foligno (PG) (IT)
(72) Inventor: Cocchioni, Pasquale, I-06034 Foligno (Perugia) (IT)
(74) Representative: Carangelo, Pierluigi

(56) References cited:
- WO-A2-2007/059888
- DATABASE GNPD [Online] MINTEL; 30 August 2013 (2013-08-30), "Deep moisture Cream Without Perfume", XP002744816, Database accession no. 2124069
- DATABASE GNPD [Online] MINTEL; 29 March 2002 (2002-03-29), "Stress Balance Facial Care", XP002744817, Database accession no. 141792
- DATABASE GNPD [Online] MINTEL; 30 April 2008 (2008-04-30), "Baby Wet Wipes", XP002744818, Database accession no. 903565

## Description

### Field of the technique of the invention

This invention relates to a cosmetic composition, in particular a composition in the form of cream or lotion, for make-up removal and a textile product, in particular a wipe, soaked in such a composition.

### State of the art

Among the cosmetic products used daily by the female population, compositions for removing make-up play an important role. Such compositions normally have the form of lotions or, sometimes, emulsions or creams. The latter are preferred for their greater consistency which promotes relaxation of the skin from which the make-up is to be removed. Very often these creams also have emollient and soothing functions and thus provide an actual skin treatment together with the cleansing action.

A first defect of these products is a certain greasiness, in particular deriving from the oily component.

In addition, given that these creams are applied by means of wipes or pads made of cellulose or synthetic material, their texture makes absorption on the textile support difficult, for which their use is not practical. WO2007059888 A2 discloses oil-in-water emulsions for impregnating and wetting hygienic tissue substrates such as make-up removal wipes, said emulsions containing a combination of non-ionic and anionic emulsifiers and oily components.

### Summary of the invention

One purpose of this invention is to provide a make-up remover composition in the form of emulsion, in particular a cream, which has low greasiness and which can be easily absorbed on a textile support such as a wipe or pad made of natural or synthetic material.

An object of this invention is therefore a make-up remover composition in the form of an oil-in-water emulsion as outlined in accompanying claims 1 to 9.

Another object of this invention is a make-up remover product, consisting of a textile backing, such as a wipe or a pad made of natural or synthetic material, soaked with the make-up remover composition of the invention, as outlined in accompanying claims 10 to 12.

Further characteristics and advantages of the process according to the invention will be evident from the following description of examples of preferred embodiments.

### Detailed description of the invention

This invention relates to a make-up remover composition in the form of an oil-in-water emulsion containing:
a) at least one hydrocarbon with C13-C30 chain,
b) an oily component comprising at least one ester of a fatty alcohol,
c) at least one alcohol with C12-C24 chain,
d) cetearyl glucoside, wherein cetearyl glucoside is contained in the composition in an amount of 0.15% to 2% by weight, on the total weight of the composition.

In certain embodiments, the at least one hydrocarbon with C13-C30 chain is contained in the composition in an amount between 0.5% and 4% by weight, preferably between 1% and 2% by weight, on the total weight of the composition.

In certain embodiments, the at least one hydrocarbon with C13-C30 chain is a branched hydrocarbon preferably selected from isododecane (2,2,4,4,6,6,8-heptamethylnonane) and isoparaffins C13-C14.

In certain embodiments, the oily component comprises:
- 1-6% by weight, preferably 2-4% by weight, on the total weight of the composition of an oil of medium polarity,
- 0.5-4% by weight, preferably 1-2% by weight, on the total weight of the composition of an oil of high polarity.

Preferred examples of medium polarity oils are: ethylhexyl stearate, ethylhexyl palmitate, C12-C15 alkyl benzoates, cetearylethyl hexanoate, isononyl isononanoate esters of fatty alcohols C4-C26 with fatty acids C8-C14.

Preferred examples of high polarity oils are: caprylic-capric triglyceride oil and sunflower oil (oil of Elantius annuum).

In certain embodiments, the alcohols with C12-C22 chain, preferably with C14-C18 chain, have a melting point > 40 °C and are contained in an amount of at least 0.75% by weight , preferably between 1% and 1.5% by weight, relative to the total weight of the composition.

Preferred examples of such alcohols are: cetyl alcohol, cetearyl alcohol, cetostearyl alcohol, arachidyl alcohol and behenyl alcohol.

Cetearyl glucoside is contained in the composition in an amount between 0.15% and 2% by weight, preferably between 0.25% and 2% by weight, more preferably between 0.5% and 1% by weight, on the total weight of the composition.

Optionally, the composition of the invention may comprise an additional emulsifier in an amount not exceeding 1% by weight on the total weight of the composition.

Preferred examples of such emulsifiers are: cetearyl olivate/sorbitan olivate, sucrose cocoate/sorbitan stearate, sorbitan stearate/sorbityl laurate, cetaeryl polyglucoside, polyglyceryl-3-methylglucose distearate, cetearyl polyglucoside and polyglyceryl-2 dipolyhydroxystearate.

The composition of the invention must have a sufficiently low viscosity to favour the imbibition of a textile support, such as a wipe or pad made of natural or synthetic material.

Therefore, in certain embodiments, the composition according to the invention has a viscosity between 1 and 1000 mPa, preferably between 10 and 200 mPa, measured by a Brookfield-type rotational viscometer, at a speed 100 rpm and a temperature of 25 °C.

The long-chain fatty alcohols are waxes and give the composition better smoothness and spreadability, in addition increasing the viscosity.

The use of cetearyl glucoside in the quantities indicated above is thus essential for obtaining of a stable oil-in-water emulsion of the desired low viscosity.

The composition of the invention has a good texture, is easily absorbed on a textile support and has scant greasiness.

The oil-in-water emulsion according to the invention can be obtained by means of a particularly advantageous process, which involves the following steps:
i) preparation by heating of a solution or suspension of cetearyl glucoside and optionally at least one additional emulsifier in a first aliquot of water;
ii) preparation by heating of a mixture of said at least one hydrocarbon with C13-C30 chain with said oily component;
iii) hot mixing of the solution or suspension according to step i) with the mixture according to step ii) obtaining a primary emulsion;
iv) mixing of said primary emulsion according to step iii) with a second aliquot of water at low temperature, obtaining said oil-in-water emulsion.

In certain embodiments, said first aliquot of water in step i) is between 10% and 35%, preferably between 18% and 28%, of the water required for the final emulsion. Consequently, the second aliquot of water of step iv) is between 90% and 65%, preferably between 82% and 72%, of the water required for the final emulsion.

In certain embodiments, step i) is conducted at a temperature between 40 °C and 90 °C, or between 55 °C and 75 °C. In certain embodiments, the mixture is stirred for a time of between 0.25 and 1.5 hours, preferably between 0.5 and 1 hour.

In certain embodiments, step ii) is conducted at a temperature between 45 °C and 90 °C, preferably between 60 °C and 75 °C.

Step iii) is preferably carried out by means of an emulsifying pump. In any case, the necessary steps will be taken to obtain an emulsion. The primary emulsion thus obtained is stirred at a temperature preferably between 40 °C and 70 °C, more preferably between 50 °C and 60 °C.

According to step iv), the second aliquot of water, corresponding to a percentage of between 90% and 65% of the total water contained in the composition, is preferably at a temperature between 7 °C and 25 °C, more preferably between 12.5 °C and 20 °C.

In certain embodiments step iv) is conducted by emulsifying the second aliquot of water and the primary emulsion for a time between 10 and 80 minutes, preferably between 25 and 50 minutes.

A further object of the invention is a cosmetic product for make-up removal comprising a textile support soaked in the cosmetic composition according to the invention.

The textile support used can typically be a wipe or pad.

The textile support according to the invention can be made from a material that is natural, such as cellulose pulp or cotton fibres, artificial (for example viscose) or synthetic (for example, polypropylene fibre).

In the case of synthetic material, the textile support of the invention will be typically made from a nonwoven fabric, composed for example of fibres of polyolefins, polyesters, polyamides, polyacrylates or similar polymers normally used in the industry.

The impregnation of the textile support with the cosmetic composition of the invention can be made according to the most common techniques used in the industry, such as spraying, coating, dipping, etc.

As previously mentioned, the invention achieves the intended purposes, by making available a make-up remover cosmetic composition with low greasiness, excellent consistency and improved impregnability of a textile support.

## Claims

1. Make-up remover composition in the form of oil-in-water emulsion containing:
a) at least one hydrocarbon with C13-C30 chain,
b) an oily component comprising at least one ester of a fatty alcohol,
c) at least one alcohol with C12-C22 chain,
d) cetearyl glucoside,
wherein cetearyl glucoside is contained in the composition in an amount between 0.15% and 2% by weight, on the total weight of the composition.

2. Composition according to claim 1, wherein the at least one hydrocarbon with C13-C30 chain is contained in the composition in an amount between 0.5% and 4% by weight, or between 1% and 2% by weight, on the total weight of the composition.

3. Composition according to claim 1 or 2, wherein the at least one hydrocarbon with C13-C30 chain is a branched hydrocarbon preferably selected from isododecane (2,2,4,4,6,6,8-heptamethylnonane) and isoparaffins C13-C14.

4. Compound according to any of claims 1 to 3, in which the oily component comprises:
- 1-6% by weight, preferably 2-4% by weight, on the total weight of the composition of an oil of medium polarity selected from the group consisting of ethylhexyl stearate, ethylhexyl palmitate, C12-C15 alkyl benzoates, cetearylethyl hexanoate, isononyl isononanoate and esters of fatty alcohols C4-C26 with fatty acids C8-C14,
- 0.5-4% by weight, preferably 1-2% by weight, on the total weight of the composition of an oil of high polarity selected from the group consisting of caprylic-capric triglyceride oil and sunflower oil (oil of *Elantius annuum*).

5. Composition according to any of claims 1 to 4, wherein the alcohols with C12-C22 chain, preferably with C14-C18 chain, have a melting point > 40 °C and are contained in an amount of at least 0.75% by weight , or between 1% and 1.5% by weight, relative to the total weight of the composition.

6. Composition according to any of claims 1 to 5, wherein the alcohols with C12-C22 chain are chosen from cetyl alcohol, cetearyl alcohol, arachidyl alcohol and behenyl alcohol.

7. Composition according to any one of claims 1 to 6, wherein cetearyl glucoside is contained in the composition in an amount between 0.5% and 1% by weight, on the total weight of the composition.

8. Composition according to any of claims 1 to 7, wherein the composition contains an additional emulsifier in an amount not exceeding 1% by weight on the total weight of the composition, said emulsifier preferably being selected from cetearyl olivate/sorbitan olivate, sucrose cocoate/sorbitan stearate, sorbitan stearate/sorbityl laurate, cetaeryl polyglucoside, polyglyceryl-3-methylglucose distearate, cetearyl polyglucoside and polyglyceryl-2 dipolyhydroxystearate.

9. Composition according to any of claims 1 to 8, wherein the composition has a viscosity between 1 and 1000 mPa, or between 10 and 200 mPa, measured by a Brookfield-type rotational viscometer, at a speed 100 rpm and a temperature of 25 °C.

10. Cosmetic product for make-up removal, comprising a textile support soaked in the cosmetic composition according to any of claims 1 to 9.

11. Cosmetic product according to claim 10, wherein said cosmetic product is a wipe or pad.

12. Cosmetic product according to claim 10 or 11, wherein the textile support is a non-woven fabric made of natural or synthetic fibre, wherein the natural fibre is preferably cellulose and wherein the synthetic fibre is preferably selected from polyolefins, polyesters, polyamides and polyacrylates.

## Patentansprüche

1. Make-up-Entferner-Zusammensetzung in Form einer Öl-in-Wasser-Emulsion, die Folgendes enthält:
a) zumindest einen Kohlenwasserstoff mit einer C13-C30-Kette,
b) eine ölhaltige Komponente, die zumindest einen Ester eines Fettalkohols aufweist,
c) zumindest einen Alkohol mit einer C12-C22-Kette,
d) Cetearylglucosid,
wobei Cetearylglucosid in der Zusammensetzung in einer Menge zwischen 0,15 Gew.-% und 2 Gew.-% des Gesamtgewichts der Zusammensetzung enthalten ist.

2. Zusammensetzung gemäß Anspruch 1, bei der der zumindest eine Kohlenwasserstoff mit C13-C30-Kette in der Zusammensetzung in einer Menge zwischen 0,5 Gew.-% und 4 Gew.-%, oder zwischen 1 Gew.-% und 2 Gew.-%, des Gesamtgewichts der Zusammensetzung enthalten ist.

3. Zusammensetzung gemäß Anspruch 1 oder 2, bei der der zumindest eine Kohlenwasserstoff mit C13-C30-Kette ein verzweigter Kohlenwasserstoff ist, der vorzugsweise aus Isododecan (2,2,4,4,6,6,8-Heptamethylnonan) und Isoparaffinen C13-C14 gewählt ist.

4. Verbindung gemäß einem der Ansprüche 1 bis 3, bei der die ölhaltige Komponente Folgendes aufweist:
- 1-6 Gew.-%, vorzugsweise 2-4 Gew.-%, des Gesamtgewichts der Zusammensetzung eines mittelpolaren Öls, das aus der Gruppe gewählt wird, die aus Ethylhexylstearat, Ethylhexylpalmitat, C12-C15-Alkylbenzoaten, Cetearylethylhexanoat, Isononylisononanoat und Estern von Fettalkoholen C4-C26 mit Fettsäuren C8-C14 besteht,
- 0,5-4 Gew.-%, vorzugsweise 1-2 Gew.-%, des Gesamtgewichts der Zusammensetzung eines hochpolaren Öls, das aus der Gruppe gewählt wird, die aus Capryl-Caprin-Triglycerid-Öl und Sonnenblumenöl (Öl des *Elantius annuum*) besteht.

5. Zusammensetzung gemäß einem der Ansprüche 1 bis 4, bei der die Alkohole mit einer C12-C22-Kette, vorzugsweise mit einer C14-C18-Kette, einen Schmelzpunkt > 40 °C aufweisen und relativ zu dem Gesamtgewicht der Zusammensetzung in einer Menge von zumindest 0,75 Gew.-%, oder zwischen 1 Gew.-% und 1,5 Gew.-%, enthalten sind.

6. Zusammensetzung gemäß einem der Ansprüche 1 bis 5, bei der die Alkohole mit C12-C22-Kette aus Cetylalkohol, Cetearylalkohol, Arachidylalkohol und Behenylalkohol gewählt sind.

7. Zusammensetzung gemäß einem der Ansprüche 1 bis 6, bei der Cetearylglucosid in der Zusammensetzung in einer Menge zwischen 0,5 Gew.-% und 1 Gew.-% des Gesamtgewichts der Zusammensetzung enthalten ist.

8. Zusammensetzung gemäß einem der Ansprüche 1 bis 7, bei der die Zusammensetzung einen zusätzlichen Emulgator in einer Menge enthält, die 1 Gew.-% des Gesamtgewichts der Zusammensetzung nicht übersteigt, wobei der Emulgator vorzugsweise aus Cetearylolivat/Sorbitanolivat, Saccharosecocoat/Sorbitanstearat, Sorbitanstereat/Sorbityllaurat, Cetaerylpolyglucosid, Polyglyceryl-3-Methylglucosedistearat, Cetearylpolyglucosid und Polyglyceryl-2-Dipolyhydroxystearat gewählt ist.

9. Zusammensetzung gemäß einem der Ansprüche 1 bis 8, bei der die Zusammensetzung eine Viskosität zwischen 1 und 1000 mPa, oder zwischen 10 und 200 mPa, die durch ein Rotationsviskosimeter nach der Brookfield-Methode gemessen wird, bei einer Geschwindigkeit von 100 UpM und einer Temperatur von 25 °C aufweist.

10. Kosmetikprodukt zur Make-up-Entfernung, das einen textilen Träger aufweist, der mit der Kosmetikzusammensetzung gemäß einem der Ansprüche 1 bis 9 getränkt ist.

11. Kosmetikprodukt gemäß Anspruch 10, wobei das Kosmetikprodukt ein Tuch oder ein Pad ist.

12. Kosmetikprodukt gemäß Anspruch 10 oder 11, bei dem der textile Träger ein Vliesstoff aus einer natürlichen oder synthetischen Faser ist, wobei die natürliche Faser vorzugsweise Cellulose ist und wobei die synthetische Faser vorzugsweise aus Polyolefinen, Polyestern, Polyamiden und Polyacrylaten gewählt ist.

## Revendications

1. Composition de démaquillant sous la forme d'une émulsion huile dans l'eau contenant :
a) au moins un hydrocarbure avec une chaîne en C13 à C30,
b) un composant huileux comprenant au moins un ester d'un alcool gras,
c) au moins un alcool avec une chaîne en C12 à C22,
d) du glucoside de cétéaryle,
dans laquelle le glucoside de cétéaryle est contenu dans la composition en une quantité entre 0,15 % et 2 % en poids, sur le poids total de la composition.

2. Composition selon la revendication 1, dans laquelle le au moins un hydrocarbure avec une chaîne en C13 à C30 est contenu dans la composition en une quantité entre 0,5 % et 4 % en poids, ou entre 1 % et 2 % en poids, sur le poids total de la composition.

3. Composition selon la revendication 1 ou 2, dans laquelle le au moins un hydrocarbure avec une chaîne en C13 à C30 est un hydrocarbure ramifié sélectionné de préférence parmi l'isododécane (2,2,4,4,6,6,8-heptaméthylnonane) et les isoparaffines en C13 à C14.

4. Composé selon l'une quelconque des revendications 1 à 3, dans lequel le composant huileux comprend :
- 1 à 6 % en poids, de préférence 2 à 4 % en poids, sur le poids total de la composition d'une huile de polarité moyenne sélectionnée dans le groupe constitué par le stéarate d'éthylhexyle, le palmitate d'éthylhexyle, les benzoates d'alkyle en C12 à C15, l'hexanoate de cétéaryléthyle, l'isononanoate d'isononyle et les esters d'alcools gras en C4 à C26 avec des acides gras en C8 à C14,
- 0,5 à 4 % en poids, de préférence 1 à 2 % en poids, sur le poids total de la composition d'une huile de polarité élevée sélectionnée dans le groupe constitué par l'huile de triglycérides capryliques-capriques et l'huile de tournesol (huile d'*Elantius annuum*).

5. Composition selon l'une quelconque des revendications 1 à 4, dans laquelle les alcools avec une chaîne en C12 à C22, de préférence avec une chaîne en C14 à C18, ont un point de fusion > 40 °C et sont contenus en une quantité d'au moins 0,75 % en poids, ou entre 1 % et 1,5 % en poids, par rapport au poids total de la composition.

6. Composition selon l'une quelconque des revendications 1 à 5, dans laquelle les alcools avec une chaîne en C12 à C22 sont choisis parmi l'alcool cétylique, l'alcool cétéarylique, l'alcool arachidylique et l'alcool béhénylique.

7. Composition selon l'une quelconque des revendications 1 à 6, dans laquelle le glucoside de cétéaryle est contenu dans la composition en une quantité entre 0,5 % et 1 % en poids, sur le poids total de la composition.

8. Composition selon l'une quelconque des revendications 1 à 7, dans laquelle la composition contient un émulsifiant additionnel en une quantité ne dépassant pas 1 % en poids sur le poids total de la composition, ledit émulsifiant étant de préférence sélectionné parmi l'olivate de cétéaryle/l'olivate de sorbitane, le cocoate de sucrose/stéarate de sorbitane, le stéarate de sorbitane/laurate de sorbityle, le polyglucoside de cétéaryle, le distéarate de polyglycéryl-3-méthylglucose, le polyglucoside de cétéaryle et le 2-dipolyhydroxystéarate de polyglycéryle.

9. Composition selon l'une quelconque des revendications 1 à 8, dans laquelle la composition a une viscosité entre 1 et 1 000 mPa, ou entre 10 et 200 mPa, mesurée par un viscosimètre rotatif de type Brookfield, à une vitesse de 100 tr/min et à une température de 25 °C.

10. Produit cosmétique pour le démaquillage, comprenant un support textile trempé dans la composition cosmétique selon l'une quelconque des revendications 1 à 9.

11. Produit cosmétique selon la revendication 10, dans lequel le produit cosmétique est une serviette ou un disque.

12. Produit cosmétique selon la revendication 10 ou 11, dans lequel le support textile est un tissu non tissé réalisé en fibre naturelle ou synthétique, dans lequel la fibre naturelle est de préférence la cellulose et dans lequel la fibre synthétique est de préférence sélectionnée parmi les polyoléfines, les polyesters, les polyamides et les polyacrylates.
